# EUROPÄISCHE PATENTANMELDUNG

(11) **EP 0 915 330 A1**
(43) Veröffentlichungstag der Anmeldung: **12.05.1999**
(21) Anmeldenummer: 98120933.1
(22) Anmeldetag: 04.11.1998
(51) Int. Cl.: G01N 15/08

(54) **Verfahren und Vorrichtung zur Prüfung der Flüssigkeitsaufnahme poröser Baustoffe**

(30) Priorität: 05.11.1997 DE 19748777
(71) Anmelder: Pleyers, Gerd, Dipl.-Ing., 52146 Würselen (DE)
(72) Erfinder: Pleyers, Gerd, Dipl.-Ing., 52146 Würselen (DE)
(74) Vertreter: Schmetz, Bruno, Dipl.-Ing.

(57) **Zusammenfassung**

Es ist ein Verfahren zur Prüfung der Flüssigkeitsaufnahme poröser Baustoffe (7) offenbart, bei dem ein definierter Kontaktbereich der Baustoffoberfläche mit einer in einer Kammer (2) befindlichen Flüssigkeit in Kontakt gebracht und die Menge der aufgenommenen Flüssigkeit und gegebenenfalls der zeitliche Verlauf der Flüssigkeitsaufnahme ermittelt werden, das dadurch gekennzeichnet ist, daß ein an den Rändern des definierten (inneren) Kontaktbereichs gelegener weiterer (äußerer) Kontaktbereich der Baustoffoberfläche mit der gleichen, jedoch in einem zweiten Kammer (4) befindlichen Flüssigkeit in Kontakt gebracht wird.

Mit diesem Verfahren ist im Vergleich zu dem bekannten Karsten'schen Verfahren eine bessere Ermittlung des Eindringverhaltens der Flüssigkeit in den Baustoff erzielbar.

Weiter offenbart ist eine Vorrichtung zur Durchführung des Verfahrens.

## Beschreibung

Die vorliegende Erfindung betrifft ein Verfahren und eine Vorrichtung zur Prüfung der Flüssigkeitsaufnahme poröser Baustoffe gemäß dem Oberbegriff des Patentspruchs 1 bzw. 2.

Die Prüfung der Flüssigkeitsaufnahme poröser Baustoffe ist unter verschiedensten Gesichtspunkten in der Baupraxis von großer Bedeutung. Nachstehend sind unterschiedlichste Anwendungsgebiete als Beispielanwendungen aufgeführt:
- Überprüfung der Schlagregendichtheit von Fassaden (Ziegel-, Putz- oder andere Untergründe). Diese kann zum einen im Rahmen der Qualitätssicherung der Ausführung herangezogen und zum anderen kann z.B. bei einem durch Schlagregen durchfeuchteten Mauerwerk auf die Ursache geschlossen werden.
- Überprüfung der Dichtheit von Beton z.B. im Rahmen der Dichtheitsprüfung eines Betons, der als Sekundärbarriere für die Lagerung von wassergefährdenden Stoffen eingesetzt wird.
- Überprüfung des Eindringvermögens von Imprägnierstoffen in poröse Baustoffe (Beton, Naturstein etc.). Hieraus kann mit Hilfe des zugänglichen Porenvolumens auf die Eindringtiefe des Imprägnierstoffes geschlossen werden.
- Ermittlung des sogenannten Wasseraufnahmekoeffizienten als Kennwert zur Einstufung von Baustoffen hinsichtlich des Wasseraufnahmeverhaltens (vgl. DIN 52617).
- Überprüfung der Dauerwirksamkeit von hydrophobierenden Imprägnierstoffen (Einsatzgebiete: Schutz und Instandsetzung von Beton und Naturstein).

Die vorgenannten Einsatzgebiete der Prüfung der Flüssigkeitsaufnahmne sind beispielhaft aufgeführt und zielen in allen Fällen darauf ab, einen spezifischen Kennwert der Kombination aus Baustoff und Flüssigkeit zu erhalten. Aus den Anwendungsbeispielen gebt hervor, daß diese Prüfung im wesentlichen vor Ort am Bauwerk stattfindet.

Es ist bekannt, das baustoffspezifische Wasseraufnahmeverhalten im Labor gravimetrisch zu bestimmen. Hierzu werden prismatische Probekörper des Baustoffs verwendet, die ein 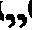 quasi eindimensionales" Eindringen der Prüfflüssigkeit (in der Regel Wasser) gewährleisten. Unter 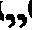 eindimensional" wird dabei ein Eindringen verstanden, daß in Richtung der tiefer gelegenen Baustoffschichten erfolgt. Das Eindringen parallel zur Baustoffoberfläche kann bei dünnen Probekörpern vernachlässigt werden bzw. wird vernachlässigt. Diese relativ exakte gravimetrische Labormethode eignet sich in vielen Fällen jedoch wegen der damit verbundenen Zerstörung der Bausubstanz durch Entnahme der Probekörper mittels z.B. Kernbohrungen nicht. Außerdem ist sie verhältnismäßig aufwendig.

Im Stand der Technik wird zur Prüfung der Flüssigkeitsaufnahme poröser Baustoffe weiter das nach seinem Erfinder benannte Karsten'sche Prüfröhrchen verwendet (vgl. Karsten, R.: Bauchemie: Für Studium und Praxis, 9. Aufl., Karlsruhe, Müller 1992 und Karsten, R.: Schlagregendichte Klinkerfassaden. In: Das Baugewerbe 43 (1963), Nr. 18, S. 1192.4-1192.7. 1963).

Dieses Vorrichtung wird seit rd. 25 Jahren in der Praxis in erster Linie am Bauwerk zur Bestimmung der Schlagregendichtheit von Fassaden eingesetzt. Bei der Vorrichtung handelt es sich um einen Glaskörper, mit dem man eine definierte Baustoffoberfläche mit z.B. Wasser beaufschlagen kann. Das Eindringverhalten über die Zeit ist durch eine entsprechende Teilung am entsprechend angeordneten Standrohr zu erfassen. Das Ergebnis kann somit in z.B. ml je definierter Beaufschlagungsfläche und Zeiteinheit angegeben werden. Es sind Ausführungen für vertikale und horizontale Baustoffoberflächen verfügbar.

Es ist weiter eine Abwandlung des Karsten'schen Prüfröhrchens bekannt geworden (vgl. DE 296 20 040 U1), mit der das Wasseraufnahmevermögen von Fassaden und Mauerwerk unter Einbeziehung eines definierten Anteils der Fugen bestimmt werden soll. Bei dieser Art der Prüfung ergeben sich jedoch Probleme aus den geometrischen Randbedingungen, die in Anlehnung an Wendler, Snethlage und Rapp (Wendler, E. ; Snethlage, R.: Der Wassereindringprüfer nach Karsten - Anwendung und Interpretation der Meßwerte. München : Bayerisches Landesamt für Denkmalpflege, 1991. - Forschungsbericht Nr. 3, S. 23-34. 1991 und Rapp, K., Wendler, E., Snethlage, R.: Zerstörungsfreie Messung der Wasseraufnahme - Verbessertes Auswerteverfahren für die Meßmethode nach Karsten. S.288, Berichte der Deutschen Mineralogischen Gesellschaft, Beih. z. Eur. J. Mineral. Vol 9, 1997, No. 1) hier kurz erläutert werden:

Benetzt man eine kreisförmige Baustoffoberfläche mit einer Flüssigkeit, so dringt diese zum einen in Form eines Zylinders und zum anderen in Form eines diesen Zylinder umgebenden, rotierenden Viertelkreis ein. Eine Eindimensionalität des Eindringverhaltens bzw. des Körpers, in den die Flüssigkeit eindringt, ist somit nicht gewährleistet. Diese ist aber für eine definierte Bestimmung des Eindringverhaltens von Flüssigkeiten in poröse Baustoffe unter Berücksichtigung der o.a. Beispielanwendungen erforderlich.

Hieraus resultiert ein weiterer, wesentlich weitreichenderer Nachteil. Oberflächenparallele Eindringvorgänge können mit dieser bekannten Vorrichtung nicht ermittelt werden. Anhand der eindringenden Flüssigkeitsmenge, die je Zeiteinheit beobachtet werden kann, ist die Beurteilung bzgl. der Eindringtiefe nicht mit ausreichender Sicherheit möglich. Insbesondere bei Voruntersuchungen zur Imprägnierfähigkeit von Baustoffen ist diese Information aber von großer Relevanz.

Ursache eines oberflächenparallelen Eindringverhaltens z.B. bei Naturstein sind Inhomogenitäten im Porensystem, die z.B. durch die steinspezifische Entstehungsgeschichte, durch Verwitterungsvorgänge, durch hohe Untergrundfeuchte etc. bedingt sein können.

Diese Problematik wurde von Wendler, Snethlage und Rapp erkannt. Abhilfe wurde mit Hilfe einer Software geschaffen, die bedingt in der Lage ist, aus der mit dem Karsten'schen Prüfröhrchen ermittelten flächenspezifischen Flüssigkeitsaufnahme je Zeiteinheit die eindimensional eingedrungene Flüssigkeit zu ermitteln.

Aus den vorliegenden Erfahrungen mit dem Karsten'schen Prüfröhrchen und der entwickelten Software geht hervor, daß
- nur bedingte Baustellentauglichkeit gegeben ist (PC-Betrieb an der Baustelle erforderlich);
- nicht jede Meßreihe mit der Software auswertbar ist (Hintergrund: Das gewählte mathematische Modell geht von idealen geometrischen Randbedingungen aus. Inhomogenitäten des Baustoffuntergrundes können nicht berücksichtigt werden);
- ein eindimensionales Eindringverhalten generell nur sehr bedingt erzielbar ist,
- oberflächenparallele Eindringvorgänge nur bedingt erfaßbar sind, und
- der notwendige Zeitaufwand durch Einsatz der notwendigen Auswertesofware für den Baustelleneinsatz einen Nachteil darstellt.

Aufgabe der vorliegenden Entwicklung ist es daher, dem Anwender ein Verfahren und eine zugehörige Vorrichtung zur Prüfung der Flüssigkeilsaufnahme von porösen Baustoffen zur Verfügung zu stellen, das das eindimensionale Eindringverhalten ausreichend genau ermittelt und oberflächenparallele Eindringvorgänge erfaßt, das baustellentauglich (geringe Abmessungen, einfache Handhabbarkeit, einfache Beurteilung der Meßergebnisse vor Ort), kostengünstig (konkurrenzfähig mit handelsüblichen Meßverfahren/-techniken), schnell (Ausgabe des Meßergebnisses unmittelbar nach Messung) und zerstörungsfrei (kein Eingriff in die Bausubstanz) ist.

Diese Aufgabe wird gelöst mit einem Verfahren gemäß Patentanspruch 1 bzw. einer Vorrichtung gemäß Patentanspruch 2.

Dadurch, daß ein an den Rändern des definierten (inneren) Kontaktereichs gelegener weiterer (äußerer) Kontaktbereich der Baustoffoberfläche mit der gleichen, jedoch in einer zweiten Kammer befindlichen Flüssigkeit in Kontakt gebracht wird, wird erzielt, daß sich die in der inneren Kammer befindliche Flüssigkeit eindimensional ausbreitet. Die wie im genannten Stand der Technik in die Randbereiche abfließende Flüssigkeit entstammt hier nicht der inneren Kammer und verfälscht so das Meßergebnis, sondern entstammt der äußeren Kammer.

Zur Durchführung des Verfahrens dient eine aufdem Karsten'schen Prüfrohrchen basierende Vorrichtung, bei der an den Rändem der ersten Flüssigkeitskammer eine zweite, mittels einer zweiten Abdichteinrichtung mit dem umgebenden (äußeren) Kontaktbereich der Baustoffoberfläche in Kontakt bringbare zweite Flüssigkeitskammer mit Steigrohr angeordnet ist.

Vorteilhafte Ausgestaltungen sind Gegenstand der Unteransprüche.

Die Erfindung wird nachstehend anhand bevorzugter Ausführugsformen unter Bezugnahme auf die beigefügten Zeichnungsfiguren erläutert, die Folgendes zeigen:
Figur 1 a) und b) zeigt eine erste Ausführungsform der Vorrichtung für vertikale Baustoffoberflächen in Vorder- und Seitenansicht;
Figur 2 a) und b) zeigt eine zweite Ausführungsform der Vorrichtung für horizontale Baustoffoberflächen in Vorder- und Seitenansicht;
Figur 3 a) und b) zeigt die Ausführungsform aus Figur 1 im Einsatz (Eindringen in die Tiefe);
Figur 4 zeigt die Ausführungsform aus Figur 1 im Einsatz (oberflächenparalleles Eindringen);
Figur 5 zeigt ein Detail der äußeren Flüssigkeitskammer; und
Figur 6 zeigt verschiedene Ausführungsformen der Steigrohre.

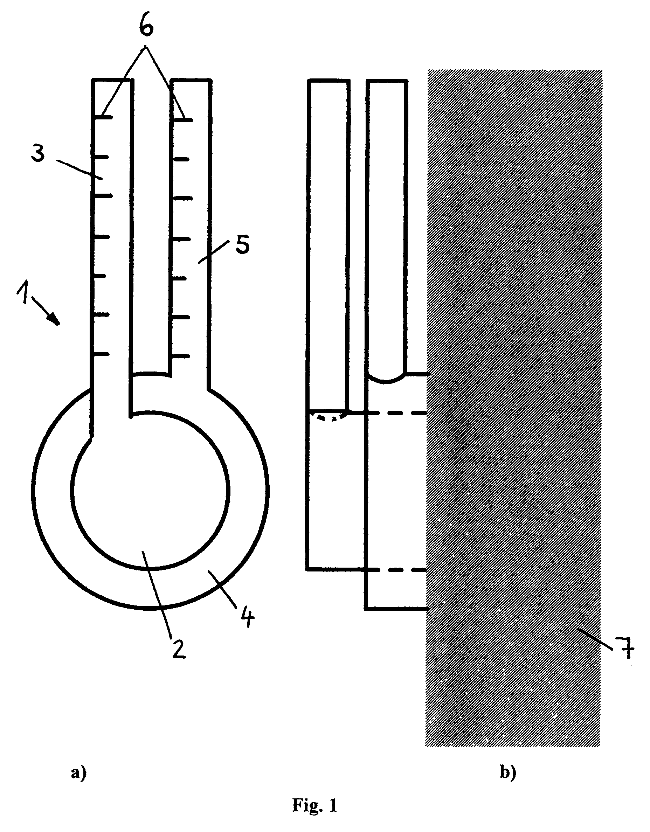

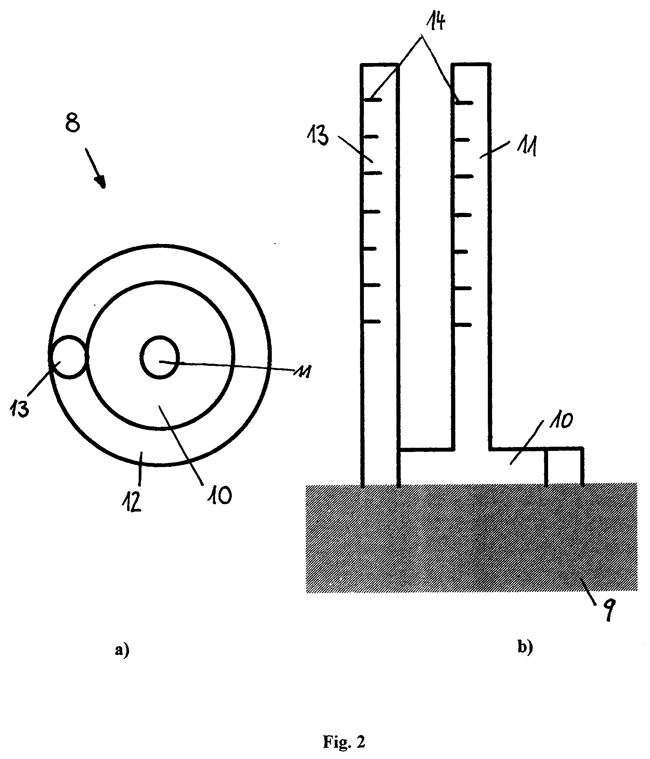

In den Figuren 1 und 2 sind zwei verschiedene Ausführungsformen der Vorrichtung zur Prüfung der Flüssigkeitsaufnahme an vertikalen und horizontalen Baustoffoberflächen dargestellt.

In Figuren 1 a) und b) ist eine bevorzugte Ausführungsform 1 der Vorrichtung für vertikale Baustoffoberflächen 7 dargestellt. Man erkennt die zylindrische innere Flüssigkeitskammer 2 mit dem zugeordneten und mit dieser in Verbindung stehenden Steigrohr 3. Um die innere Flüssigkeitskammer 2 ist die äußere hohlzylindrische Flüssigkeitskammer 4 angeordnet. Auch diese weist ein zugeordnetes und mit dieser in Verbindung stehendes Steigrohr 5 auf. Die Grundflächen der Flüssigkeitskammern 2 und 4 sind senkrecht zu den Längsachsen der Steigrohre 3 und 5. Beide Steigrohre 3 und 5 weisen eine Maßteilung 6 z.B. in kg/m², ml/m² oder ml auf. In Figur 1 b) ist erkennbar, wie die Vorrichtung 1 mit der Baustoffoberfläche 7 in Kontakt gebracht wird.

In Figuren 2 a) und b) ist eine bevorzugte Ausführungsform 8 der Vorrichtung 1 für horizontale Baustoffoberflächen 9 dargestellt. Man erkennt die zylindrische innere Flüssigkeitskammer 10 mit dem zugeordneten und mit dieser in Verbindung stehenden Steigrohr 11. Um die innere Flüssigkeitskammer 10 ist die äußere hohlzylindrische Flüssigkeitskammer 12 angeordnet. Auch diese weist ein zugeordnetes und mit dieser in Verbindung stehendes Steigrohr 13 auf. Die Flächennormalen der Grundflächen der Flüssigkeitskammern 10 und 12 sind senkrecht zu den Längsachsen der Steigrohre11 und 13. Beide Steigrohre 11 und 13 weisen eine Maßteilung 14 z.B. in kg/m², ml/m² oder ml auf. In Figur 2 b) ist erkennbar, wie die Vorrichtung 8 mit der Baustoffoberfläche 9 in Kontakt gebracht wird.

Im Folgende wird die Anwendung anhand der Ausführungsform 1 weiter erläutert, wobei die Ausführungen für die Ausführungsform 8 entsprechend gelten.

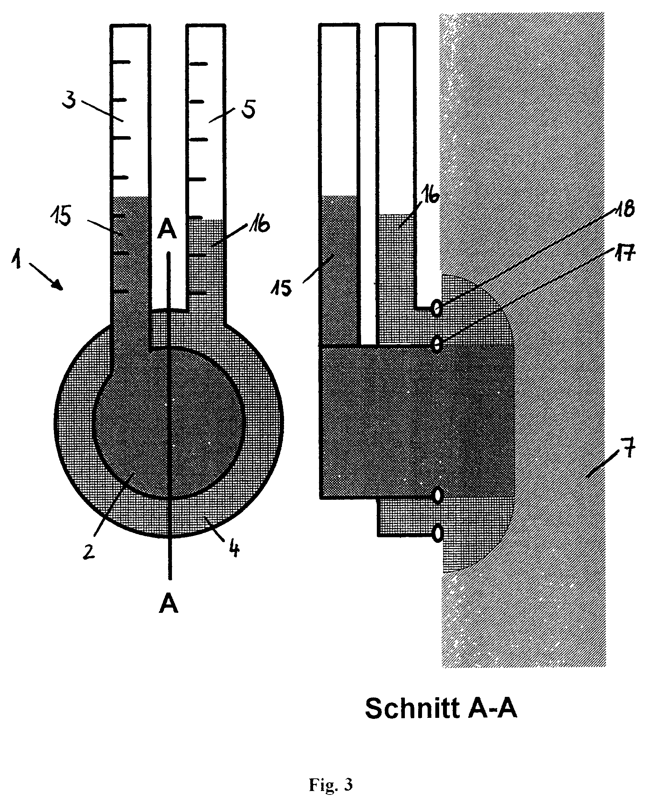

In Figuren 3 a) und b) ist wiederum die Vorrichtung 1 entsprechend Figuren 1 a) und b) gezeigt. Bei der Figur 3 handelt es sich allerdings nicht um eine Seitenansicht wie in Figur 1 b), sondern um eine Schnittansicht entlang Linie A-A aus Figur 3 a), wobei zur Verdeutlichung die beiden Steigrohre 3 und 5 zusätzlich in Seitenansicht eingefügt sind.

In Figur 3 ist zu erkennen, das sich zwischen der inneren Flüssigkeitskammer 2 und der äußeren Flüssigkeitskammer 4 eine Dichtung 17, z.B. ein O-Ring aus Neopren befindet, der die beiden Flüssigkeitskammern 2 und 4 gegeneinander abdichtet. Am äußeren Rand der äußeren Flüssigkeitskammer 4 befindet sich eine weitere Dichtung 18, z.B. Plastilinkit, die die äußere Flüssigkeitskammer 4 gegen die Umgebung abdichtet und die Vorrichtung 1 an der Baustoffoberfläche 7 festhält. Wie in der Detailfigur 5 gezeigt, kann die äußere Flüssigkeitskammer 4 zu diesem Zweck auch einen Flansch 19 aufweisen, der dem Plastilinkit 18 als Widerlager dient.

Nach dem Befestigen der Vorrichtung 1 an der Baustoffoberfläche wird in die innere Flüssigkeitskammer 2 ein erster Teil 15 und in die äußere Flüssigkeitskammer 4 ein zweiter Teil 16 einer Flüssigkeit, z.B. Wasser eingefüllt. Der erste Teil 15 der Flüssigkeit dringt daraufhin in Form eines Zylinders und der zweite Teil 16 der Flüssigkeit dringt in Form eines rotierenden Viertelkreises (ein Viertel eines Torus) in den angrenzenden Baustoff 7 ein. Die Eindimensionalität des Eindringens aus der inneren Flüssigkeitskammer 2 kann besonders eindrucksvoll demonstriert werden, wenn in die Flüssigkeitskammern 2 und 4 unterschiedlich gefärbte Flüssigkeiten eingefüllt werden und man diese in einen Probekörper eindringen läßt. Schneidet man anschließend den Probekörper auf, findet sich eine exate Trennlinie zwischen den beiden Farben.

Zur Auswertung dient die Menge und der zeitliche Verlauf der aufgenommenen Menge des ersten Teils der Flüssigkeit 15, die eindimensional eindringt. Die üblichen Verfälschungen durch nicht-eindimensionales Eindringen an den Randbereichen werden durch den in diesen Bereich ständig nachströmenden zweiten Teil 16 der Flüssigkeit kompensiert. Während des Meßvorgangs muß allerdings sichergestellt werden, daß die äußere Flüssigkeitskammer 4 nicht trockenläuft, da ansonsten auch wieder aus der inneren Flüssigkeitskammer Flüssigkeit 15 in nicht-eindimensionaler Weise in den umliegenden Baustoff eindringt.

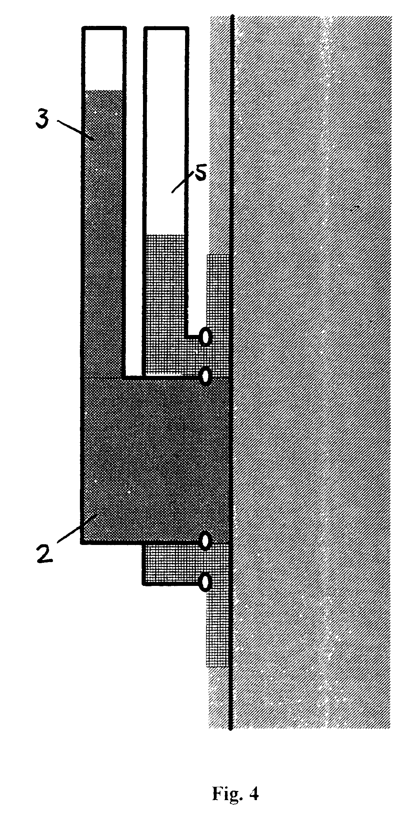

Wie in Figur 4 dargestellt, ist es mit der Vorrichtung erstmalig auch möglich, exakte Aussagen über oberflächenparallele oder oberflächennahe Eindringvorgänge zu gewinnen. Findet ein Flüssigkeitstransport nämlich hauptsächlich in diesem Bereich statt, wird der Flüssigkeitsstand im Steigrohr 3 nach einer Weile nicht mehr abfallen, da die unter der inneren Flüssigkeitskammer 2 befindliche Oberfläche bereits gesättigt wohingegen der Flüssigkeitsstand im Steigrohr 5 weiter fällt, da die Flüssigkeit in die oberflächlichen Randbereiche abfließt.

Zur Dimensionierung der vorgestellten Vorrichtungen ist Folgendes anzumerken: Grundsätzlich sind verschiedene Dimensionierungen (unterschiedlich große Vorrichtungen, unterschiedliche Verhältnisse von innerer und äußerer Flüssigkeitskammer, unterschiedliche Steigrohrdurchmesser usw.) möglich. Bei bestimmten Anwendungen können aber bestimmte Größen bzw. Größenverhältnisse vorteilhaft sein.

Zur besseren Beurteilung der Qualität des 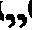 oberflächenparallelen Saugens" sollte die Grundfläche (= innerer Kontaktbereich) der ersten Flüssigkeitskammer und die Grundfläche (= äußerer Kontaktbereich) der zweiten Flüssigkeitskammer flächengleich sein.

Um den zeitlichen Verlauf der Flüssigkeitsaufnahme des inneren und äußeren Bereichs besser beurteilen zu können, ist es weiter empfohlen, die Volumina der ersten und zweiten Flüssigkeitskammer einschließlich ihrer Steigrohre gleich zu halten. Besonders bevorzugt ist es, wenn dabei die Durchmesser der Steigrohre der ersten und zweiten Flüssigkeitskammer gleich sind.

Für unterschiedlich stark saugende Baustoffe ist es sinnvoll, komplette Vorrichtungen mit unterschiedlichen Steigrohrdurchmessern zur Verfügung zu haben. Je nach Saugverhalten des Baustoffes kann die Sensibilität der Messung angepaßt werden, z.B. 1 ml Einteilung statt 5 ml Einteilung.

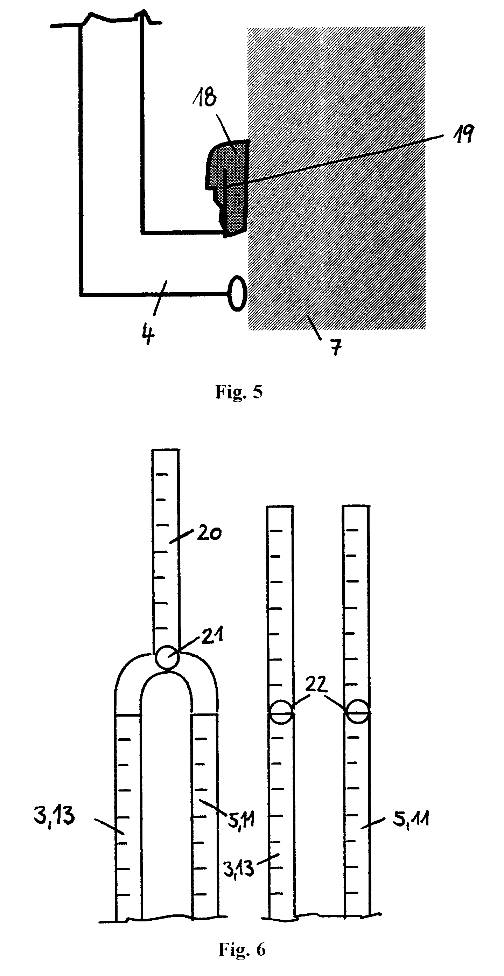

In Figuren 6 a) und b) sind zwei Varianten der Vorrichtung gezeigt, die sowohl für horizontales als auch für vertikales Eindringen geeignet ist. Sie betrifft die gleichzeitige Befüllung der Steigrohre 3 und 5 mit entsprechenden Flüssigkeitsmengen. In Figur 6 a) sind beide Steigrohre 3 und 5 über ein Ventil 21 an eine über ihnen angeordnetes drittes Steigrohr 20 angeschlossen. Nach dessen Befüllung wird das Ventil 21 geöffnet, wodurch gleichzeitig die innere und äußere Flüssigkeitskammer 2 und 4 befüllt wird. In Figur 6 b) ist gezeigt, daß auch jedes Steigrohr 3 und 5 ein Ventil 22 aufweisen kann. Nach Befüllung der oberen Teile der Steigrohre 3 und 5 werden beide Ventile 22 gleichzeitig geöffnet, wodurch wiederum die innere und äußere Flüssigkeitskammer 2 und 4 gleichzeitig befüllt wird.

Die vorgestellten Vorrichtung sind bevorzugt aus Glas hergestellt. Es ist jedoch auch möglich, diese aus z.B. lösemittelbeständigem Kunststoff auszubilden. Zur Kontrolle sollte dieser transparent sein.

Mit Hilfe des Verfahrens bzw. der Vorrichtung ist es also möglich, das Eindringverhalten von Flüssigkeiten in den Baustoff zerstörungsfrei mit ausreichender Genauigkeit zu kontrollieren. Zudem sind oberflächenparallele Eindringvorgänge zu erkennen. Außerdem ist durch entsprechende Teilungsbeschriftung des Steigrohres der inneren Flüssigkeitskammer unmittelbar auf den jeweiligen Wasseraufnahmekoeffizienten des Baustoffes zu schließen. Bei Geltung des sogenannten 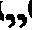 √t -Gesetzes" ist der Wasseraufnahmekoeffizient (Dimension: kg/m²·√h) des Baustoffes bereits nach 15 min zu bestimmen. Hierzu ist lediglich die abgelesene eingedrungene Wassermenge mit dem Faktor 4 zu multiplizieren.

### BEZUGSZIFFERNLISTE

- 1: Vorrichtung zur Prüfung der Flüssigkeitsaufnahme (für vertikale Messung)
- 2: Innere Flüssigkeitskammer
- 3: Steigrohr
- 4: Äußere Flüssigkeitskammer
- 5: Steigrohr
- 6: Maßteilung
- 7: Baustoff
- 8: Vorrichtung zur Prüfung der Flüssigkeitsaufnahme (für horizontale Messung)
- 9: Baustoff
- 10: Innere Flüssigkeitskammer
- 11: Steigrohr
- 12: Äußere Flüssigkeitskammer
- 13: Steigrohr
- 14: Maßteilung
- 15: Innerer Teil der Flüssigkeit
- 16: Äußerer Teil der Flüssigkeit
- 17: Dichtung
- 18: Dichtung
- 19: Flansch
- 20: Steigrohr
- 21: Ventil
- 22: Ventil

## Patentansprüche

1. Verfahren zur Prüfung der Flüssigkeitsaufnahme poröser Baustoffe, bei dem ein definierter Kontaktbereich der Baustoffoberfläche mit einer in einer Kammer befindlichen Flüssigkeit in Kontakt gebracht und die Menge der in einer bestimmten Zeit aufgenommenen Flüssigkeit oder der zeitliche Verlauf der Flüssigkeitsaufnahme ermittelt wird,
**dadurch gekennzeichnet, daß**
ein an den Rändern des definierten Kontaktbereichs gelegener weiterer äußerer Kontaktbereich der Baustoffoberfläche mit der gleichen, jedoch in einer zweiten Kammer befindlichen Flüssigkeit in Kontakt gebracht wird.

2. Vorrichtung, insbesondere zur Durchführung des Verfahrens nach Anspruch 1, mit einer ersten Flüssigkeitskammer mit Steigrohr, die mittels einer ersten Abdichteinrichtung mit einem definierten Kontaktbereich der Baustoffoberfläche in Kontakt bringbar ist
**dadurch gekennzeichnet, daß**
an den Rändern der ersten Flüssigkeitskammer (2; 10) eine mittels einer zweiten Abdichteinrichtung (18) mit einem den definierten Kontaktbereich umgebenden äußeren Kontaktbereich der Baustoffoberfläche in Kontakt bringbare zweite Flüssigkeitskammer (4; 12) mit Steigrohr (5; 13) angeordnet ist.

3. Vorrichtung nach Anspruch 2, dadurch gekennzeichnet, daß die erste Flüssigkeitskammer (2; 10) zylindrisch und die zweite Flüssigkeitskammer (4; 12) hohlzylindrisch ausgebildet ist.

4. Vorrichtung nach Anspruch 2 oder 3, dadurch gekennzeichnet, daß die dem definierten Kontakbereich entsprechende Grundfläche der ersten Flüssigkeitskammer (2; 10) und die dem äußeren Kontaktbereich entsprechende Grundfläche der zweiten Flüssigkeitskammer (4; 12) flächengleich sind.

5. Vorrichtung nach einem der Ansprüche 2 bis 4, dadurch gekennzeichnet, daß die Volumina der ersten und zweiten Flüssigkeitskammer (2, 4; 10, 12) einschließlich ihrer Steigrohre (3, 5; 11, 13) gleich sind.

6. Vorrichtung nach einem der Ansprüche 2 bis 5, dadurch gekennzeichnet, daß die Durchmesser der Steigrohre (3, 5; 11, 13) der ersten und zweiten Flüssigkeitskammer (2, 4; 10, 12) gleich sind.

7. Vorrichtung nach einem der Ansprüche 2 bis 6, dadurch gekennzeichnet, daß die Steigrohre (3, 5; 11, 13) eine Maßteilung (6; 14) aufweisen.

8. Vorrichtung nach einem der Ansprüche 2 bis 7, dadurch gekennzeichnet, daß die erste Abdichteinrichtung (17) eine zwischen der ersten und zweiten Flüssigkeitskammer (2, 4, 10, 12) dauerhaft eingesetzte Dichtung wie z.B. ein O-Ring ist.

9. Vorrichtung nach einem der Ansprüche 2 bis 8, dadurch gekennzeichnet, daß die Grundflächen der Flüssigkeitskammern (2, 4, 10, 12) senkrecht zu den Längsachsen der Steigrohre (3, 5; 11, 13) sind.

10. Vorrichtung nach einem der Ansprüche 2 bis 8, dadurch gekennzeichnet, daß die Flächennormalen der Grundflächen der Flüssigkeitskammern (2, 4, 10, 12) senkrecht zu den Längsachsen der Steigrohre (3, 5; 11, 13) sind.

11. Vorrichtung nach einem der Ansprüche 2 bis 10, dadurch gekennzeichnet, daß sie aus Glas oder transparentem Kunststoff gefertigt ist.

12. Vorrichtung nach einem der Ansprüche 2 bis 11, dadurch gekennzeichnet, daß die Steigrohre (3, 5; 11, 13) über ein Ventil (21) an ein über ihnen angeordnetes drittes Steigrohr (20) angeschlossen sind.

13. Vorrichtung nach einem der Ansprüche 2 bis 11, dadurch gekennzeichnet, daß die Steigrohre (3, 5; 11, 13) jeweils ein Ventil (22) aufweisen.

14. Vorrichtung nach einem der Ansprüche 2 bis 13, dadurch gekennzeichnet, daß die zweite Flüssigkeitskammer (4; 12) an ihrer Außenwandung einen Flansch (19) zur Aufnahme von Dichtmassen (18) aufweist.
